# EUROPEAN PATENT SPECIFICATION

(11) **EP 3 319 581 B1**
(45) Date of publication and mention of the grant of the patent: **18.06.2025**
(21) Application number: 16744543.6
(22) Date of filing: 21.07.2016
(51) Int. Cl.: A61K 6/16, A61K 6/17, A61K 6/20, A61K 6/77, A61K 6/836, A61K 6/887, A61P 1/02, C08F 222/10

(54) **DENTAL COMPOSITIONS**
DENTALE ZUSAMMENSETZUNGEN
COMPOSITIONS DENTAIRES

(43) Date of publication of application: 16.05.2018
(73) Proprietor: Queen Mary University of London, London E1 4NS (GB)
(72) Inventor: HILL, Robert, London E1 4NS (GB); SHAHID, Saroash, London E1 4NS (GB); PATEL, Mangala, London E1 4NS (GB)
(74) Representative: Bassil, Nicholas Charles
(86) International application number: PCT/IB2016/054358
(87) International publication number: WO 2017/006302

(56) References cited:
- US-A- 4 310 584
- US-A1- 2006 241 205
- US-A1- 2009 088 515
- US-A1- 2013 281 601
- FERRACANE J L: "Current trends in dental composites", CRITICAL REVIEWS IN ORAL BIOLOGY AND MEDICINE, CRC PRESS, BOCA RATON, FL, US, vol. 6, no. 4, 1 January 1995 (1995-01-01), pages 302 - 318, XP009146590, ISSN: 1045-4411, DOI: 10.1177/10454411950060040301
- FRANKLIN P ET AL: "Reinforcement of poly(methyl methacrylate) denture base with glass flake", DENTAL MATERIALS, ELSEVIER, AMSTERDAM, NL, vol. 21, no. 4, 1 April 2005 (2005-04-01), pages 365 - 370, XP027760531, ISSN: 0109-5641, [retrieved on 20050401]
- DATABASE REGISTRY [online] CHEMICAL ABSTRACTS SERVICE, COLUMBUS, OHIO, US; 12 December 2000 (2000-12-12), XP002762151, Database accession no. 308066-75-3

## Description

### Field of the invention

The present invention relates to dental compositions for use in a method of treating or preventing dentine hypersensitivity or caries.

### Background

Tooth decay or caries is the most prevalent disease in the Western World and costs patients and public health authorities billions of dollars every year. Tooth decay is caused by bacteria in plaque metabolizing sugars and producing acids, which dissolve the apatite crystal phase of enamel and dentine. The bacteria also produce proteases that degrade the protein structure of the tooth. Once the enamel is lost the decay spreads rapidly into the less corrosion resistant dentine and bacteria can spread through rapidly via the dentinal tubules. Once the protein structure is lost the carious lesion cannot be restored by the natural remineralisation processes in the mouth and surgical intervention is required by the dentist.

Enamel can also be lost by the action of acidic foods such as fruits, wine, etc. Loss of enamel results in the exposure of dentine tubules leading to dentine hypersensitivity. Dentine hypersensitivity is a relatively common painful condition believed to affect up to 40% of the adult population. It is defined as "short, sharp pain arising from exposed dentine in response to stimuli, typically thermal, cold, evaporative, tactile, osmotic or chemical which cannot be ascribed to any other form of dental defect or pathology". Up to 40% of people suffer from dentine hypersensitivity and usually resort to the use of sensitivity-treating toothpastes commonly available over the counter. These toothpastes temporarily block the tubules thereby reducing sensitivity. The effect is short-lasting and not very effective in cases with severe enamel loss.

One method of protecting teeth from erosion and treating or preventing caries is the application of a dental coating such as a varnish. Varnishes are applied over the tooth surface and form a temporary layer thereby protecting the enamel. Varnishes may also contain additional agents such as fluoride which are known to be effective at treating or preventing caries. However, unless the application of the varnish results in an aesthetically pleasing tooth surface, patients are unwilling to opt for this method of treatment. For example, most fluoride varnishes are yellow or opaque white and are therefore unable to provide the desired aesthetic effect.

Dental prostheses are usually used in the oral cavity for prolonged periods of time. As a result, biofilms, such as a dental plaque, can form on the prosthetic surface leading to oral disorders for example stomatitis and inflammation of the oral mucosa.

Conventional composite resins consist of particles of ceramic or glass dispersed in a resin matrix. They have an isotropic microstructure and properties. Whilst they offer improved abrasion and wear resistance compared to unfilled resins they still exhibit significant wear in the mouth. The softer resin component is worn away during mastication largely by a three body wear process involving two tooth surfaces and small silica particles from foodstuffs. After a period of mastication ceramic/glass particles become exposed on the surface and are plucked from the surface.

There is therefore a need for dental coating materials that can combine high functionality with aesthetic appeal.

US 2006/241205 A1 relates to filler materials for the use in dental composite materials. The document describes dental restorations comprising glass flakes and a polymeric matrix precursor composition. In all examples according to the invention, the resin content in the composition is below 20% by weight.

### Summary of the invention

The present inventors have surprisingly found that a composition comprising glass flakes with specific dimensions shows improved hardness and wear resistance. The composition also provides a coating that is aesthetically pleasing. This is due to a matching of the refractive indices of the resin and glass flakes.

Accordingly, the invention provides a dental composition for use in a method of treating or preventing dentine hypersensitivity or caries according to claim 1. The dependent claims describe preferred embodiments.

### Detailed description

Glass flakes: In an embodiment of the invention the glass flakes have a thickness in the range of between 1 and 10 microns and an aspect ratio in the range of between 14:1 and 90:1. Aspect ratio is the ratio of the length to the thickness of the glass flakes. The thickness is from 0.5 to 10 microns, for example, the thickness is from 1 to 10 microns, is from 0.5 to 5, or is from 0.5 to 2 microns.

In a preferred embodiment the glass flakes have an aspect ratio of between 14:1 and 30:1. In a more preferred embodiment the aspect ratio is between 20:1 and 40:1.

The present inventors have found that glass flakes with an aspect ratio of greater than 14:1 self-align, whilst glass flakes with an aspect ratio less than 14:1 do not self-align.

Accordingly, the invention provides a dental composition comprising glass flakes having a thickness in the range of between 0.5 and 10 microns (for example of between 1 and 10 microns) and an aspect ratio in the range of between 14:1 and 90:1 wherein upon application to a tooth surface the glass flakes in the composition align substantially parallel to the surface.

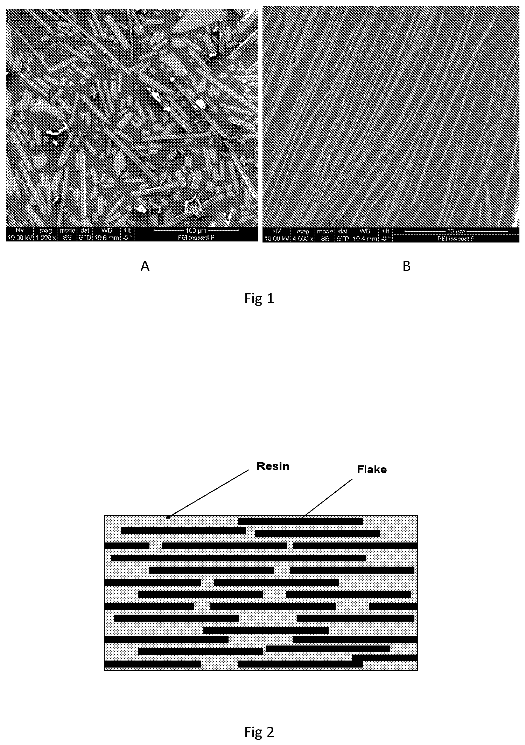

The term "substantially parallel" as used herein means that the glass flakes have a distinct orientation relative to the surface that is not random and favours one direction as demonstrated by figure 1.

Figure 2 shows a schematic aligned flake glass composite. On abrasive wear the softer resin at the near surface will be worn away leaving effectively a hard wear-resistant glass surface. Flake glass coatings therefore have excellent abrasive wear resistance compared to conventional particulate composites providing the flake particles are aligned to the surface. Without alignment of the flake the advantages using flake will be lost.

One of the surprising aspects found during the course of the studies is that the incorporation of aligned flakes has a dramatic effect on improving the efficiency of the light curing. It is thought the aligned flake particles act as physical barriers to oxygen diffusion into the resin thereby preventing the formation of an oxygen inhibited layer. Oxygen is known to be a powerful radical scavenger and to inhibit the polymerization at the surface resulting in a softer surface layer. Incorporation of flake glass eliminates this effect and results in a much harder well polymerized surface.

The tooth surface may be the surface of a natural tooth, a restored tooth for example in the case of decay or a prosthetic tooth. Also included are tooth surfaces which have been previously coated with a dental coating such as a varnish or a veneer.

The weight fraction of the glass flakes in the compositions of the invention is between 10%-40%. In a preferred embodiment, the weight fraction is 20%-40%. In a more preferred embodiment the weight fraction is 30%.

The glass flakes may be prepared from any suitable glass. For example, alkali (A) glass made from soda lime silicate (silicon dioxide, sodium oxide, calcium oxide optionally with one or more of magnesium oxide and aluminium oxide), alkali resistant (AR) glass made from zirconium silicates, corrosive resistant (C) glass made with calcium borosilicates, low dielectric constant (D) glass made with borosilicates, alkali free highly electrically resistive (E) glass, electrically corrosion resistant (ECR) glass made with aluminium-calcium borosilicates, reinforced (R) glass made with calcium aluminosilicates, high strength (S) glass made with magnesium aluminosilicates, or S-2 Glass^{®} with improved strength properties. The glass used may be selected according to the desired properties of the compositions of the invention.

The glass flakes may be prepared from an electrically corrosion resistant (ECR) glass, such as a glass comprising silicon dioxide containing one or more of the following compounds selected from the group consisting of potassium oxide (K₂O), boron trioxide (B₂O₃), zinc oxide (ZnO), sodium oxide (Na₂O, magnesium oxide (MgO), calcium oxide (CaO), aluminium oxide (Al₂O₃) and titanium oxide (TiO₂).

For example, the electrically corrosion resistant may be composed of silicon dioxide (SiO₂), potassium oxide (K₂O), boron trioxide (B₂O₃), zinc oxide (ZnO), sodium oxide (Na₂O, magnesium oxide (MgO), calcium oxide (CaO), aluminium oxide (Al₂O₃) and titanium oxide (TiO₂).

The glass flakes may be suitably milled after the glass is formed. The particle size of the glass flakes may suitably be less than 1000 microns, for example 1000 to 5 microns, such as 500 to 5 microns, or 300 to 50 microns. In a sample of glass flakes, the particle size distribution may be 10% or less of glass flakes in the range of from 1000 to 300 microns, 65% or less of glass flakes in the range of from 300 to 50 microns, and 25% or less of glass flakes in the range of less than 50 microns.

In one embodiment, the electrically corrosion resistant glass may have the following composition:

**Table 1**

| **ECR glass component** | **Amount (% by weight)** |
|---|---|
| silicon dioxide (SiO₂) | 64-70% |
| potassium oxide (K₂O) | 0-3% |
| boron trioxide (B₂O₃) | 2-5% |
| zinc oxide (ZnO) | 1-5% |
| sodium oxide (Na₂O) | 8-13% |
| magnesium oxide (MgO) | 1-4% |
| calcium oxide (CaO) | 3-7% |
| aluminium oxide (Al₂O₃) | 3-6% |
| titanium oxide (TiO₂) | 0-3% |

The glass flakes are pre-treated and coated with a silane compound, for example 3-aminopropyltriethoxy silane, vinyl trimethoxy silane, γ-glycidoxypropyltrimethoxy silane or methacryloxypropyltrimethoxy silane.

### Resin

The comprises a vinyl type resin, such as acrylic type resins, styrene type resins and the like. The vinyl resin comprises a monomer selected from 4- methacryloxyethyl trimellitic acid and its anhydride, bisphenol type epoxy acrylates and their oligomers, urethane dimethacrylates, methyl acrylate, methyl methacrylate, ethyl methacrylate, butyl methacrylate, polyethyleneglycol dimethacrylates, 2,2-bis(p-2'-hydroxy-3'-methacryloxy propoxyphenyl) propane, 2-Hydroxyethyl methacrylate, Triethylene glycol dimethacrylate, tetrahydrofurfuryl methacrylate, Bis[2-(methacryloyloxy)ethyl] phosphate, 2,2-bis(4-methacryloxy polyethoxy phenyl) propane, acrylonitril, vinyl acetate, 2-cyano acrylic acid esters, styrene and divinyl benzene. These vinyl monomers can be employed alone or in any mixture thereof.

In a preferred embodiment the resin is a methacrylate resin. In a more preferred embodiment the resin is a difunctional methacrylate thermoset resin. Thermoset resins set or harden by chemical reaction, often generating heat in the process and cannot be melted or manipulated once set. The term "difunctional" means that the resin has two functional groups.

In an embodiment of the invention the glass flakes and resin have a refractive index difference of less than 0.03 for example 0.02, 0.01 or 0.005.

The resin is present in the dental composition in amounts of from 50 weight percent to 90 weight percent.

In an embodiment the composition comprises at least one containing at least one acid group, particularly preferably 1 to 4 acid groups. Preferred acid groups are carboxylic acid, sulfonic acid, phosphonic acid and/or phosphoric acid groups. Compounds which contain carboxylic acid, phosphonic acid and/or phosphoric acid as acid group are particularly preferred. Compounds with more than one acid group can contain different acid groups or preferably identical acid groups.

### Photoinitiator system

One of the surprising aspects found during the course of the studies is that the incorporation of aligned flakes has a dramatic effect on improving the efficiency of the light curing. It is thought the aligned flake particles act as physical barriers to oxygen diffusion into the resin thereby preventing the formation of an oxygen inhibited layer. Oxygen is known to be a powerful radical scavenger and to inhibit the polymerization at the surface resulting in a softer surface layer. Incorporation of flake glass eliminates this effect and results in a much harder well polymerized surface.

"Photoinitiator system" refers to a molecule which absorbs visible or ultraviolet light, forming an excited state which can then either fragment into free radical functional groups (type I photoinitiator) capable of initiating polymerization of the monomers, or alter a second molecule by abstracting a hydrogen from the second molecule and converting the second molecule into a free radical capable of initiating polymerisation (type II photoinitiator).

In an embodiment the composition of the invention comprises a photoinitiator system. Photoinitiator systems include a mono or diketone photoinitiator together with a suitable donor compound or accelerator. In an embodiment, the photoinitiator system comprises a tertiary amine (for example ethyl-4-dimethylamino benzoate, dihydroxyethyl-para-toluidine, 2-dimethylaminoethyl methacrylate) and a photosensitiser (for example camphorquinone, 2,4,6-Trimethylbenzoyl-diphenylphosphine oxide and 1-Phenyl 1,2-propanedione (PPD)).

Any suitable photoinitiator that dissociates to form an initiating species may be employed. Preferably, the photoinitiator is effective in the visible light spectrum range. The activation wavelength of the photoinitiator will depend on the type of photoinitiator selected but may range from about 360 nm to about 520 nm, particularly from about 400 nm to 500 nm. Examples of photoinitiators include diketone type initiators and its derivatives and acylphosphine oxide type initiators.

In a preferred embodiment the photoinitiator can be cured by the application of light within one minute. For example camphorquinone. The photoinitiator is present in an amount from about 0.05% to about 1.0% by weight, such as from about 0.08% to about 0.5% by weight or from about 0.1% to about 0.25% by weight of the dental composition.

### Other additives

The dental compositions may comprise stabilisers such as, but not limited to, butylated hydroxyaniso!e (BHA), butylated hydroxytoluene (butyihydroxytoluene, BHT), propyl gallate (propyl 3,4,5- trihydroxybenzoate), tert-butylhydroquinone (TBH% tertiary butylhydroquinone), calcium propanoate (or calcium propionate), from liquid phenolic compounds such as mixed tocopherols (tocopherols, Vitamin E), rosemary extract, the liquid phenolic acid oregano oil (origanum oil), vegetable oil, from the quinoline-based antioxidant liquid ethoxyquin, from the acid-based antioxidant calcium propanoate (or calcium propionate), and from derivatives or mixtures thereof.

### Applications

Numerous applications are envisaged for the dental compositions disclosed herein. For example, the composition may be used to provide a wear resistant surface for teeth to protect against enamel erosion. In another application, the composition may be used to provide relief from dentine hypersensitivity by forming a protective layer on the tooth surface. In a further application, the composition may further comprise a fluoride releasing agent so that upon application to a tooth surface, it forms a coating on the tooth to protect against bacteria.

Accordingly, in one embodiment the dental composition is a dental coating. In another embodiment the dental composition is a dental varnish.

According to the invention, the dental composition is for use in a method of treating or preventing dentine hypersensitivity or caries.

In an embodiment the invention provides a sealant. Sealants are applied to the surface of a tooth in order to provide long-term protection against dental caries caused by the accumulation of bacteria. The bacteria in plaque produce acids that eat into the tooth, eventually causing cavities to form therein. Pits and fissures may develop in the surface of a tooth within which bacteria tend to accumulate. Sealants are commonly used to fill the pits and fissures in the surface of a tooth. The compositions of the invention can be used as sealants over composite restorations to prevent composite material from micro-leaking at the margins and contacts between the restoration and tooth. The compositions provide an adhesive seal against micro-leakage of composite material so that the margins of the restoration are well protected.

Uses of the invention as described above therefore relate to methods of treating or preventing dentine hypersensitivity or dental caries. The compositions may be used as protective agents in order to seal a tooth from exposure to the external environment in the mouth.

References herein to tooth or teeth are interchangeable unless the context specifies otherwise. Administration of the compositions of the invention to a tooth include direct application, as well as the use of an instrument to apply the composition to the tooth. The compositions may be applied as an amorphous form or sealant coating, or as a pre-formed implant, patch or cover.

Preferred features of the second and subsequent aspects of the invention are as for the first aspect *mutatis mutandis.*

### Figures

Figure 1 shows the microstructure of a composite made with a flake particles with a low aspect ratio of 14:1 (1A) and high aspect ratio (20:1).
Figure 2 shows a schematic aligned flake glass composite.
Figure 3 shows flake orientation relative to aspect ratio.
Figure 4 shows calcium release from Enamelin-coated and non-coated teeth.

### Examples

### Example 1: Formulation of composition

Formulation of Resin: Resin formulation is given in table 1 below. All ingredients were mixed together by stirring continuously at room temperature in a dark room to prevent curing of the resin. Urethane dimethacrylates and 2-Hydroxyethyl methacrylate, can be replaced with any other methacrylate such as bisphenol A glycidyl methacrylate, poly(propylene glycol) dimethacrylate, Triethylene glycol dimethacrylate, Siloranes). 4-META can be replaced by other carboxylate functional monomers and also by HEMA Phosphate and other phosphate functional monomers such as 2-dimethylaminoethyl methacrylate

**Table 2**

| **Monomer** | **Percentage (wt)** |
|---|---|
| Urethane-di-methacrylate (UDMA) | 64.55% |
| Hydroxyethyl Methacrylate (HEMA) | 34.39% |
| 4- META | 0.83% |
| Camphorquinone (CQ) | 0.11% |
| Dimethyl p toluidine | 0.12% |

Glass flakes: Glass Flakes (GF) were obtained from Glassflake Ltd, Leeds UK as milled glass flakes product GF100M.

Silylation of Flake: The silylation was conducted by immersing glass flakes (GF) into silane coupling agent [3-Tri methoxysilyl Methacrylate Propylene (MPS)] of Sigma- Aldrich, UK under control environment. The ratio of GF and silane agent was maintained at 0.3 and 0.7 respectively. The GF were mixed with resin on magnetic stirrer at room temperature (23°C) or 2 h in order to able GF particles to be more incorporate with resin. The samples were then placed into vacuum oven (Touson Mercer- Altrincham, England) under dynamic conditions at 40°C for 2h to allow the acetone to completely remove. Later the samples were washed with methanol (Sigma Aldrich, UK) to remove the unreacted resin and vacuum dried for 2h.

Incorporation of flakes in Resin: The resultant 10g of resin was mixed with 4.2g of silylated GF for 1h to achieve the uniform distribution of GF into resin. The obtained material was denoted as "Enamelin".

### Example 2: Flake glass alignment

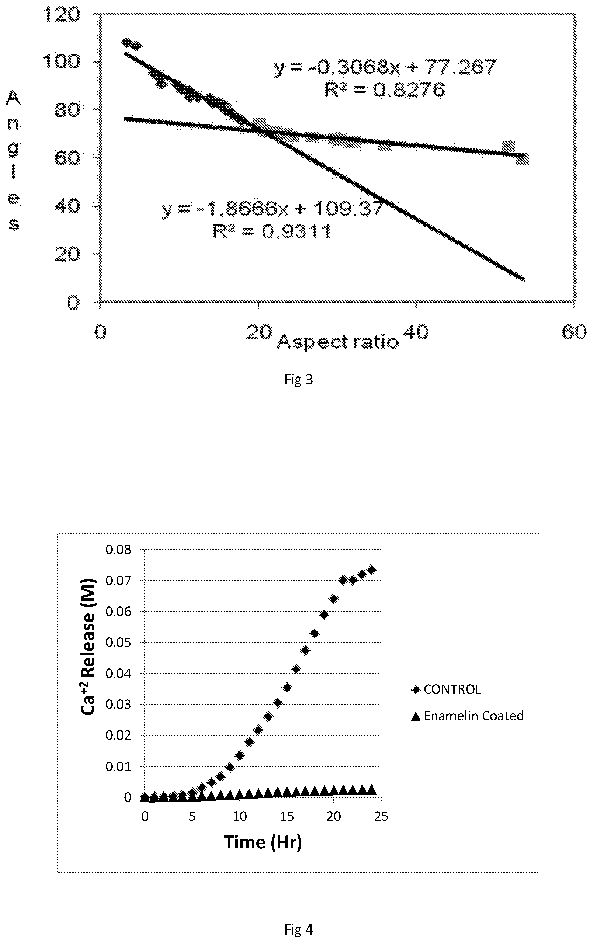

Uo et al. Journal of the Ceramic Society of Japan 118 425-7 (2010) have investigated the use of flake glass particles in dental composites however the aspect ratio of the flakes is low and the microstructure of the composite is isotropic. Compositions of the invention are anisotropic and align parallel to the surface. Figure 1a shows a scanning electron micrograph of a flake glass composite with an aspect ratio of 14:1 whilst Figure 1b with an aspect ratio > 20:1. The composite with a low aspect ratio is largely isotropic whilst the high aspect ratio composite is highly anisotropic. Figure 3 indicates that an aspect ratio greater than 14:1 is critical for the parallel alignment of the flakes. Flake glass coatings of the invention offer a potential replacement for enamel because the flake glass particles can be aligned parallel to the surface of the tooth. Alignment of the flakes can provide improved resistance to abrasive wear in the mouth.

### Example 3: Adhesion with Human tooth

This example tests the tooth adhesion of a flake glass filled methacrylate tooth coat Enamelin, and a commercially available nano silica filled methacrylate dental varnish Equia Coat (GC Corp). 12 non-carious first molars were selected from the Tissue Bank at Barts and the London School of Medicine and Dentistry. The teeth were sectioned mid-coronally using a diamond saw. The sectioned teeth were embedded in embedding resin (ClaroCit, Strauers) so that the cut dentine surface was exposed. After the acrylic resin had set hard, the exposed surfaces were acid-etched using 35% phosphoric acid solution for 20 seconds and then washed with copious amounts of water. The material, either Enamelin or Equia coat, was packed in cylindrical moulds, which were then placed on top of the tooth surface and light cured for 40seconds. 6 specimens were produced in this manner for both the materials. The specimens were stored in deionised water at 37°C for 24 hours. After 24 hours the samples were taken out from water and the Shear Bond Strength Test was performed by securing the specimens in a mounting jig and a sharp straight-edge chisel attached to the Instron cross-head was used to apply a shearing force at a rate of 0.05 mm/min.

Mean Values for tooth adhesion for Enamelin were 5.680±2.27MPa whereas for Equia Coat these were 4.63±0.705MPa. The results indicate that Enamelin has superior chemical adhesion with the organic and inorganic phases in the tooth.

### Example 4: Adhesion with Conventional Glass lonomer Cement

This example compares adhesion of a flake glass filled methacrylate dental coat Enamelin, and a commercially available nano silica filled methacrylate dental varnish to a conventional glass ionomer cement Fuji IX (GC Corp). GC Fuji IX capsules were mixed according to manufacturer's instructions and then packed into Teflon mould measuring 10mm in diameter and 1mm thickness. The cements were covered with an acetate sheet and allowed to set for 1 hour at 37°C and 100% relative humidity. The discs were then embedded in resin (ClaroCit, Strauers) with the flat surface exposed. Enamelin and Equia coat were then bonded on to the discs using the method described in Example 1. 6 samples were produced for the both the materials. Thereafter the samples were stored in deionised water at 37°C for 24 hours. After 24 hours the samples were taken out from water and the Shear Bond Strength Test was performed as described in example 1. The mean bond strength for adhesion with glass ionomer cement were 6.57±2.96MPa for Enamelin and 1.94±1.11MPa for Equia coat. Results indicate Enamelin has a superior bonding with conventional glass ionomer cements.

### Example 5: Adhesion with light cured resin modified glass ionomer cement

This example compares adhesion of a flake glass filled methacrylate dental coat Enamelin, and a commercially available nano silica filled methacrylate dental varnish with a resin modified glass ionomer cement Fuji II LC (GC Corp). GC Fuji II LC capsules were mixed according to manufacturer's instructions and then packed into mould measuring 10mm in diameter and 1mm thickness. The discs (n=12) were light cured for 20s using dental curing light. The discs were then embedded in resin (ClaroCit, Strauers) with the flat surface exposed. Enamelin and Equia coat were then bonded on to the discs using the method described in Example 1. Six samples were produced for both the materials. Thereafter the samples were stored deionised water at 37°C for 24 hours. After 24 hours the samples were removed from the water and the Shear Bond Strength Test was performed as described in example 1. The mean bond strength for adhesion with resin modified glass ionomer cement were 12.50±4.55MPa for Enamelin and 11.76±3.15MPa for Equia coat. Results indicate Enamelin has a superior bonding with conventional glass ionomer cements.

### Example 6: Vickers Hardness Test

Equia coat and Enamelin were packed into cylindrical moulds measuring 5mm (L) x2mm (D) and were then light cured for 20 s from both ends using a dental curing light. Six samples for each material were prepared. The samples were embedded in mounting resin with the flat surface exposed. The prepared samples were ground with the silicon carbide grid papers (Buehler-Met) ranges from 400, 600, 1,000, 2,400, 4,000 and finally polished with the 1µ aluminium oxide (MetPrep Ltd) paste for final smooth surface. The Vickers micro hardness test is used to assess the depth of penetration of a diamond into the material surface in order to determine its hardness. This test was carried out using a Shimadzu Micro hardness Tester, type- M by applying a load of 300g for 10 seconds. Hardness is calculated by dividing the force with the cross sectional area of the indent. Enamelin gave an average hardness of 76.68±2.96 where Equia Coat had a lower hardness of 29.35±5.68. The results show that Enamelin provides a harder coating than Equia Coat and is thus likely to be more durable in the mouth.

### Example 7: Tooth bush wear Resistance Test

Caries free human teeth (1st & 2nd premolars) - were sectioned longitudinally into two equal halves using a diamond saw. The sectioned teeth (n=6) were stored in sodium hypochlorite solution at 37°C until use. The sectioned teeth were embedded in acrylic resin so that the cut enamel and dentine surfaces were exposed. The exposed surfaces were then polished with 3µm, 0.25µ and 0.1µm diamond polishing pastes. (Kemet International Ltd, Maidstone, UK).

Enamelin (n=3) and Equia Coat (n=3) was applied using dental brushes to the prepared polished tooth samples and then light cured for 30s using a dental curing light for 30 sec. Prior to subjecting the samples to wear profilometric analysis was conducted using computerized Incise dental scanner (Renishaw, UK), with scanning interval of 0.05mm along with the speed of 500 mm/min.

Colgate Whitening Tooth paste with an RDA value of 145 (highly abrasive) was selected for the wear study. 2ml of the toothpaste was applied to the sample surfaces which were then individually placed in troughs. Tooth brush heads with flat bristles were lowered onto the samples under a constant load of 200g and 20, 000 cycles of brushing were performed using the automated equipment. After every 5,000 cycles the 2ml of distilled water along with the 2mm length toothpaste was re-applied. Wear was quantified using the profilometer again using the same parameters as described previously. Enamelin showed a wear of 0.074±0.003mm whereas Equia coat had a much higher wear of 0.129±0.002mm. This suggests Enamelin is more resistant to wear as compared to the tested commercial product. This complements the finding of the previous example as harder coating would be expected to provide a better wear resistance.

### Example 8: Protective coating against acid dissolution

Non-carious first molars were selected from the Tissue Bank at Barts and the London School of Medicine and Dentistry. The teeth were sectioned using a diamond saw to remove the tooth roots. The obtained crowns were fully coated with Enamelin using a dental brush and then light cured for 20 seconds using a LED dental light. The coated teeth were then placed in 0.1M acetic acid solution (pH 4.5). Release of calcium ions was measured over a period of 24 hours using Calcium Ion Selective Electrode (Nico 2000 Ltd, UK). Non coated crowns were used as control. These were also immersed in acetic acid of the same concentration and pH.

The results for the calcium release from coated and non-coated samples are given in figure 4. Results indicate that tooth crowns coated with Enamelin showed negligible Calcium release when compared to non-coated samples. This indicated that Enamelin forms an effective barrier against acid dissolution of teeth.

## Claims

1. A dental composition for use in a method of treating or preventing dentine hypersensitivity or caries, wherein the dental composition comprises glass flakes and a resin wherein:
the glass flakes and resin have a refractive index difference of less than 0.04;
the glass flakes are coated with a silane and have a thickness in the range of between 0.5 and 10 microns and an aspect ratio in the range of between 14:1 and 90:1;
the resin is a vinyl resin comprising a monomer selected from 4-methacryloxyethyl trimellitic acid and its anhydride, bisphenol type epoxy acrylates and their oligomers, urethane dimethacrylates, methyl acrylate, methyl methacrylate, ethyl methacrylate, butyl methacrylate, polyethyleneglycol dimethacrylates, 2,2-bis(p-2'-hydroxy-3'-methacryloxy propoxyphenyl) propane, 2-hydroxyethyl methacrylate, triethylene glycol dimethacrylate, tetrahydrofurfuryl methacrylate, bis[2-(methacryloyloxy)ethyl] phosphate, 2,2-bis(4-methacryloxy polyethoxy phenyl) propane, acrylonitril, vinyl acetate, 2-cyano acrylic acid esters, styrene and divinyl benzene; and
the weight fraction of glass flakes in the dental composition is from 10% to 40% and the weight fraction of resin in the dental composition is from 50% to 90%.

2. A dental composition for use according to claim 1, wherein the method comprises administration of the composition to a tooth.

3. A dental composition for use according to claim 1, wherein the composition is applied as a sealant over a composite restoration.

4. A dental composition for use according to any one of claims 1 to 3 wherein the aspect ratio is in the range of between 20:1 and 40:1.

5. A dental composition for use according to any one of claims 1 to 4 wherein the weight fraction of the glass flakes is between 20% and 40%.

6. A dental composition for use according to any one of claims 1 to 5 wherein the resin is a methacrylate resin.

7. A dental composition for use according to any one of claims 1 to 6 wherein the monomer is selected from urethane dimethacrylate, methyl acrylate, methyl methacrylate, ethyl methacrylate, butyl methacrylate, polyethyleneglycol dimethacrylate, 2-hydroxyethyl methacrylate, triethylene glycol dimethacrylate, tetrahydrofurfuryl methacrylate, or a mixture thereof.

8. A dental composition for use according to any one of claims 1 to 7 wherein the resin is a dimethacrylate resin.

9. A dental composition for use according to claim 8 wherein the resin comprises a hydrophilic monomer.

10. A dental composition for use according to any one of claims 1 to 9 further comprising a photoinitiator system.

11. A dental composition for use according to any one of claims 1 to 10 further comprising a carboxylic acid or phosphate functional monomer.

12. A dental composition for use according to any one of claims 1 to 11 further comprising a fluoride releasing agent.

## Patentansprüche

1. Dentalzusammensetzung zur Verwendung in einem Verfahren zur Behandlung oder Vorbeugung von Dentinüberempfindlichkeit oder Karies, wobei die Dentalzusammensetzung Glasflakes und ein Harz umfasst, wobei:
die Glasflakes und das Harz eine Brechungsindexdifferenz von weniger als 0,04 aufweisen;
die Glasflakes mit einem Silan beschichtet sind und eine Dicke im Bereich zwischen 0,5 und 10 Mikron und ein Aspektverhältnis im Bereich zwischen 14:1 und 90:1 aufweisen;
es sich bei dem Harz um ein Vinylharz handelt, das ein Monomer umfasst, das aus 4-Methacryloxyethyltrimellitsäure und ihrem Anhydrid, Epoxyacrylaten vom Bisphenoltyp und ihren Oligomeren, Urethandimethacrylaten, Methylacrylat, Methylmethacrylat, Ethylmethacrylat, Butylmethacrylat, Polyethylenglykoldimethacrylaten, 2,2-Bis(p-2'-hydroxy-3'-methacryloxypropoxyphenyl)propan, 2-Hydroxyethylmethacrylat, Triethylenglykoldimethacrylat, Tetrahydrofurfurylmethacrylat, Bis[2-(methacryloyloxy)ethyl]phosphat, 2,2-Bis(4-methacryloxypolyethoxyphenyl)propan, Acrylnitril, Vinylacetat, 2-Cyanoacrylsäureestern, Styrol und Divinylbenzol ausgewählt ist; und
der Gewichtsanteil an Glasflakes in der Dentalzusammensetzung 10 % bis 40 % beträgt und der Gewichtsanteil an Harz in der Dentalzusammensetzung 50 % bis 90 % beträgt.

2. Dentalzusammensetzung zur Verwendung nach Anspruch 1, wobei das Verfahren die Verabreichung der Zusammensetzung an einen Zahn umfasst.

3. Dentalzusammensetzung zur Verwendung nach Anspruch 1, wobei die Zusammensetzung als Versiegelungsmittel über einer Kompositrestauration aufgebracht wird.

4. Dentalzusammensetzung zur Verwendung nach einem der Ansprüche 1 bis 3, wobei das Aspektverhältnis im Bereich zwischen 20:1 und 40:1 liegt.

5. Dentalzusammensetzung zur Verwendung nach einem der Ansprüche 1 bis 4, wobei der Gewichtsanteil der Glasflakes zwischen 20 % und 40 % liegt.

6. Dentalzusammensetzung zur Verwendung nach einem der Ansprüche 1 bis 5, wobei es sich bei dem Harz um ein Methacrylatharz handelt.

7. Dentalzusammensetzung zur Verwendung nach einem der Ansprüche 1 bis 6, wobei das Monomer aus Urethandimethacrylat, Methylacrylat, Methylmethacrylat, Ethylmethacrylat, Butylmethacrylat, Polyethylenglykoldimethacrylat, 2-Hydroxyethylmethacrylat, Triethylenglykoldimethacrylat, Tetrahydrofurfurylmethacrylat oder einer Mischung davon ausgewählt ist.

8. Dentalzusammensetzung zur Verwendung nach einem der Ansprüche 1 bis 7, wobei es sich bei dem Harz um ein Dimethacrylatharz handelt.

9. Dentalzusammensetzung zur Verwendung nach Anspruch 8, wobei das Harz ein hydrophiles Monomer umfasst.

10. Dentalzusammensetzung zur Verwendung nach einem der Ansprüche 1 bis 9, die ferner ein Photoinitiatorsystem umfasst.

11. Dentalzusammensetzung zur Verwendung nach einem der Ansprüche 1 bis 10, die ferner ein carbonsäure- oder phosphatfunktionelles Monomer umfasst.

12. Dentalzusammensetzung zur Verwendung nach einem der Ansprüche 1 bis 11, die ferner ein Fluorid freisetzendes Mittel umfasst.

## Revendications

1. Composition dentaire pour une utilisation dans un procédé de traitement ou de prévention de l'hypersensibilité de la dentine ou des caries, dans laquelle la composition dentaire comprend des flocons de verre et une résine dans laquelle :
les flocons de verre et la résine ont une différence d'indices de réfraction inférieure à 0,04 ;
les flocons de verre sont revêtus par un silane et ont une épaisseur dans la plage de 0,5 à 10 microns et un rapport d'aspect dans la plage de 14:1 à 90:1 ;
la résine est une résine vinylique comprenant un monomère choisi parmi l'acide 4-méthacryloxyéthyl trimellitique et son anhydride, les époxyacrylates de type bisphénol et leurs oligomères, les diméthacrylates d'uréthane, l'acrylate de méthyle, le méthacrylate de méthyle, le méthacrylate d'éthyle, le méthacrylate de butyle, les diméthacrylates de polyéthylèneglycol, le 2,2-bis(p-2'-hydroxy-3'-méthacryloxy propoxyphényl) propane, le méthacrylate de 2-hydroxyéthyle, le diméthacrylate de triéthylène glycol, le méthacrylate de tétrahydrofurfuryle, le phosphate de bis[2-(méthacryloyloxy) éthyle], le 2,2-bis(4-méthacryloxypolyéthoxyphényl) propane, l'acrylonitrile, l'acétate de vinyle, les esters d'acide 2-cyano acrylique, le styrène et le divinylbenzène ; et
la fraction pondérale de flocons de verre dans la composition dentaire est de 10 % à 40 % et la fraction pondérale de résine dans la composition dentaire est de 50 % à 90 %.

2. Composition dentaire pour une utilisation selon la revendication 1, dans laquelle le procédé comprend l'administration de la composition à une dent.

3. Composition dentaire pour une utilisation selon la revendication 1, dans laquelle la composition est appliquée en tant qu'agent d'étanchéité sur une restauration composite.

4. Composition dentaire pour une utilisation selon l'une quelconque des revendications 1 à 3, dans laquelle le rapport d'aspect est dans la plage comprise entre 20:1 et 40:1.

5. Composition dentaire pour une utilisation selon l'une quelconque des revendications 1 à 4, dans laquelle la fraction pondérale des flocons de verre est comprise entre 20 % et 40 %.

6. Composition dentaire pour une utilisation selon l'une quelconque des revendications 1 à 5, dans laquelle la résine est une résine de méthacrylate.

7. Composition dentaire pour une utilisation selon l'une quelconque des revendications 1 à 6, dans laquelle le monomère est choisi parmi le diméthacrylate d'uréthane, l'acrylate de méthyle, le méthacrylate de méthyle, le méthacrylate d'éthyle, le méthacrylate de butyle, le diméthacrylate de polyéthylèneglycol, le méthacrylate de 2-hydroxyéthyle, le diméthacrylate de triéthylèneglycol, le méthacrylate de tétrahydrofurfuryle ou un mélange de ceux-ci.

8. Composition dentaire pour une utilisation selon l'une quelconque des revendications 1 à 7, dans laquelle la résine est une résine de diméthacrylate.

9. Composition dentaire pour une utilisation selon la revendication 8, dans laquelle la résine comprend un monomère hydrophile.

10. Composition dentaire pour une utilisation selon l'une quelconque des revendications 1 à 9, comprenant en outre un système de photoinitiateur.

11. Composition dentaire pour une utilisation selon l'une quelconque des revendications 1 à 10, comprenant en outre un monomère fonctionnalisé par un acide carboxylique ou un phosphate.

12. Composition dentaire pour une utilisation selon l'une quelconque des revendications 1 à 11, comprenant en outre un agent de libération de fluorure.
